# EUROPEAN PATENT SPECIFICATION

(11) **EP 3 908 349 B1**
(45) Date of publication and mention of the grant of the patent: **21.08.2024**
(21) Application number: 19817315.5
(22) Date of filing: 12.12.2019
(51) Int. Cl.: A61M 5/20, A61M 5/32, A61M 5/50, A61M 5/315

(54) **MEDICAMENT DELIVERY DEVICE**
MEDIKAMENTENVERABREICHUNGSVORRICHTUNG
DISPOSITIFS D'ADMINISTRATION DE MÉDICAMENTS

(30) Priority: 11.01.2019 US 201962791443 P; 08.04.2019 EP 19167823
(43) Date of publication of application: 17.11.2021
(73) Proprietor: SHL Medical AG, 6302 Zug (CH)
(72) Inventor: KRIS, Dane, 6302 Zug (CH)
(86) International application number: PCT/EP2019/084867
(87) International publication number: WO 2020/143991

(56) References cited:
- WO-A1-2009/095701
- WO-A1-2011/005177
- WO-A1-2016/055295
- US-A1- 2013 324 925
- US-A1- 2018 318 514

## Description

### TECHNICAL AREA

The present invention relates to a medicament delivery device and in particular a medicament delivery device that is designed for improved and facilitated assembly.

### BACKGROUND OF INVENTION

Many medicament delivery device on the market are devices having a number of automatic or semi-automatic features in order to facilitate the use for a user, and in particular when used for self-administration.

The automatic or semi-automatic features entail a number of components and elements that are to interact with each other in order to provide the desired function. Because many medicament delivery devices for self-administration are of generally elongated tubular design, a number of these components and elements are positioned coaxial inside or outside each other.

This may pose problems when a medicament delivery device of that design is to be assembled, and in particular if the assembly process is to be automated or at least semi-automated. The components and elements then have to be placed inside each other in certain order and in many cases temporarily connected to each other in order to e.g. hold a drive spring in a tensioned position. Some examples are disclosed in WO2016/055295A1, WO2011/005177A1, US2013/0324925A1, WO2009/095701A1, and US2018/0318514A1.

### BRIEF DESCRIPTION OF INVENTION

In the present disclosure, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

The aim of the present application is to remedy the drawbacks of the state of the art medicament delivery devices. This aim is solved with a medicament delivery device according to the features of the independent patent claim. Preferable embodiments form the subject of the dependent patent claims.

According to one aspect, a medicament delivery device comprises a housing, which housing is arranged to accommodate a medicament container. The medicament delivery device further comprises a power pack arranged to exert a force on said medicament container for expelling a dose of medicament. Further the power pack comprises a plunger rod, a drive spring arranged to exert a force on the plunger rod, an actuator coaxial with the plunger rod and arranged with holding elements for releasibly holding the plunger rod with the drive spring in an energized state, an actuator sleeve coaxial with said actuator and arranged to interact with the holding elements, the actuator sleeve being movable in a longitudinal direction in relation to the actuator. Moreover, a medicament delivery member guard is comprised in the medicament delivery device protruding from the proximal end of the housing and movable in the longitudinal direction, the medicament delivery member guard being connected to the actuator sleeve, and a manually operable activator arranged at a distal end of the housing movable in said longitudinal direction and operably connected to said actuator for releasing said plunger rod.

In addition a locking arrangement is arranged for releasibly locking the actuator to the actuator sleeve, such that movement of the medicament delivery member guard in a distal direction when pressing the medicament delivery device against an injection site causes the actuator sleeve, the actuator and the plunger rod to move in a distal direction, and that subsequent manual movement of the activator causes a movement of the plunger rod and the actuator in relation to the actuator sleeve in a proximal direction, causing a release of the plunger rod by the actuator.

According to a further aspect, the locking arrangement comprises a flexible member on the actuator provided with a proximally directed stop surface, on which stop surface a distally directed surface of the actuator sleeve will act during movement in the distal direction, preventing relative movement of the actuator in relation to the actuator sleeve in a proximal direction, and wherein the activator is arranged with ledges acting on the flexible member, removing the contact between the stop surface and the surface of the actuator sleeve, enabling movement of the actuator and the plunger rod.

Moreover, the activator may further comprise a proximally directed member, designed to act on a distal end of the plunger rod for enabling movement in the proximal direction of the plunger rod and the actuator. The medicament delivery device may further comprise a driver operably connected between the plunger rod and a medicament container holder such that movement of the plunger rod in the proximal direction will cause the driver and thus the medicament container holder in a proximal direction, causing a penetration operation.

According to a further aspect, the driver may have a releasable connection to the plunger rod such that the plunger rod is released from the driver when a penetration operation has ended, enabling the plunger rod to continue in the proximal direction, acting on a stopper of the medicament and causing an injection operation. In this regard, the releasable connection may comprise protrusions on an inner surface of the driver cooperating with recesses on the plunger rod. Further, the plunger rod may be arranged with a longitudinal groove aligned with the recess, in which recess the protrusion of the driver may slide during the injection operation.

According to a further aspect, the power pack may comprise a guide rod provided with fastening elements at its distal end, which fastening elements engage cut-outs on the actuator, and wherein the drive spring is arranged between a proximal end wall of the plunger rod and the fastening elements. In addition, the holding elements of the actuator may comprise two proximally directed arms that are flexible in the generally radial direction, which arms are arranged with inwardly directed arc-shaped protrusions engaging recesses in the plunger rod, and which arm are arranged with outwardly directed surfaces engaging an inner surface of the actuator sleeve.

These and other aspects of, and advantages with, the present invention will become apparent from the following detailed description of the invention and from the accompanying drawings.

### BRIEF DESCRIPTION OF DRAWINGS

In the following detailed description of the invention, reference will be made to the accompanying drawings, of which
Fig. 1 is an exploded view of a medicament delivery device according to the present application,
Figs. 2-13 and 16-18 are detailed view of components comprised in the medicament delivery device of Fig. 1,
Figs. 14-15 are cross-sectional view of the medicament delivery device of Fig. 1 in an initial stage,
Fig. 19 is a detailed cross-sectional view of a safety features of the medicament delivery device of Fig. 1, and
Figs. 20-26 are different functional views of the medicament delivery device of Fig. 1.

### DETAILED DESCRIPTION OF THE INVENTION

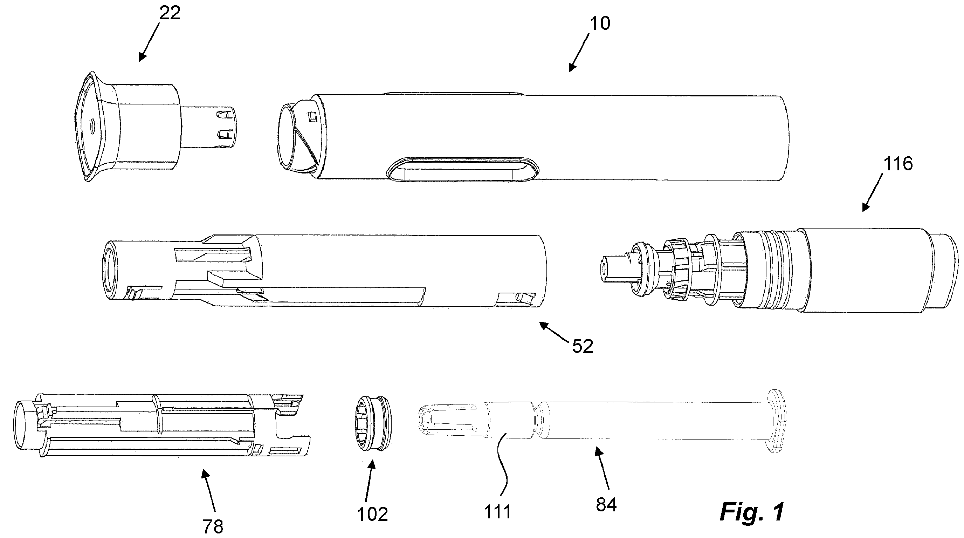

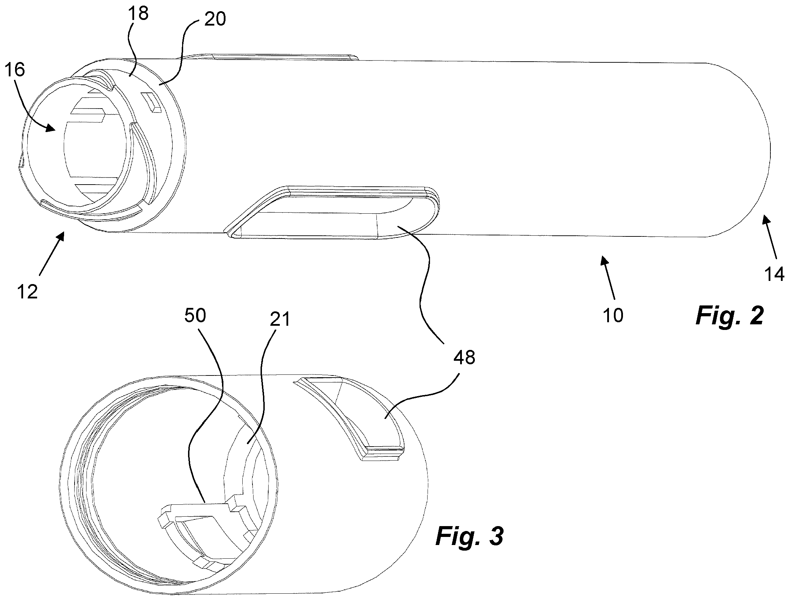

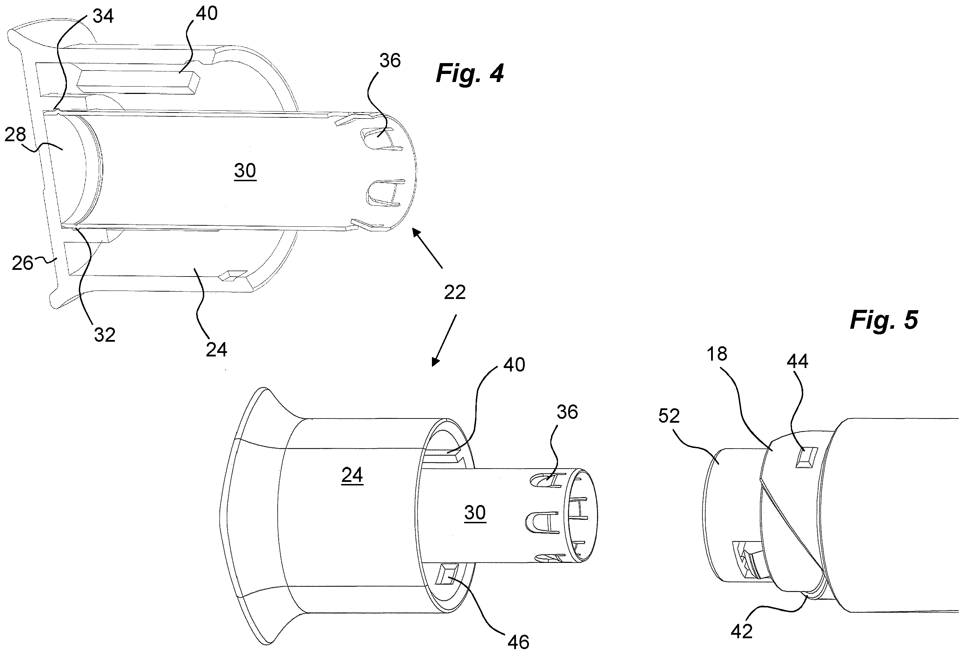

The medicament delivery device shown in the drawings comprises a generally tubular housing 10 having a proximal end 12 and a distal end 14, Fig. 1. The proximal end 12 of the housing 10 is arranged with a central passage 16, Fig. 2. An outer proximal area 18 of the housing 10 is provided with somewhat lesser diameter, providing a proximally directed circumferential ledge 20. Further, a distally directed ledge 21 is provided inside the housing 10, Fig. 3. The proximal area 18 is arranged to accommodate a distal area of a safety cap 22 having a generally tubular body 24 and a transversal end wall 26, Fig. 4. The distal surface of the end wall 26 is arranged with a generally tubular seat 28 into which a generally tubular medicament delivery member shield remover 30 is placed. The medicament delivery member shield remover 30 is held in place by an outwardly directed circumferential ledge 32 of the medicament delivery member shield remover fitting into an inwardly directed groove 34 of the seat 28. The distal end of the medicament delivery member shield remover 30 is arranged with proximally directed, inclined gripping tongues 36 that are designed to grip into an outer surface of a medicament delivery member guard 38 as will be described.

The inner surface of the body 24 of the safety cap 22 is arranged with longitudinally extending ledges 40. Some of the ledges 40 extend to a distal end surface of the safety cap 22 and some ledges 40 end a distance from the end surface of the safety cap 22. The ends of the ledges 40 are designed to interact with inclined proximally directed ledges 42 on the proximal area 18 of the housing 10, the function of which will be described below. The proximal area 18 of the housing 10 is further arranged with outwardly directed protrusions 44, Fig. 5, which protrusions 44 are designed to fit into corresponding recesses 46 on the inner surface of the safety cap 22. The housing 10 of the medicament delivery device is further arranged with windows 48, Fig. 2, on the side surface thereof, placed opposite each other. Around the windows 48 on the inner surface of the housing, longitudinally extending guide surfaces 50 are arranged, Fig. 3, the function of which will be described below.

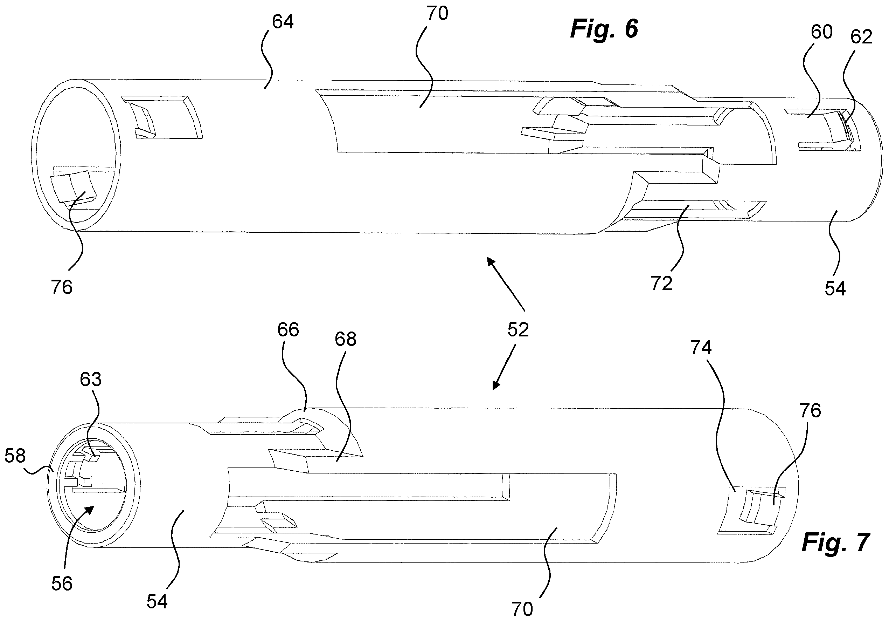

The medicament delivery device is further arranged with a medicament delivery member guard 52, Figs. 6 and 7, having a tubular proximal end portion 54 extending through the passage 16 of the housing 10. The proximal end of the medicament delivery member guard 52 is arranged with a central passage 56, which passage 56 has an inwardly directed circumferential ledge 58, forming a contact surface when the medicament delivery device is pressed against a dose delivery site. The proximal end portion 54 is further provided with a proximally directed tongue 60, where the free ends of the tongue 60 is provided with an outwardly directed wedge-shaped protrusion 62. The free end of the tongue 60 is also arranged with inwardly directed protrusions 63. The medicament delivery member guard 52 is further designed with a main, tubular body 64 having a diameter somewhat larger than the proximal end portion 54. Transition surfaces 66 are formed between them. Further longitudinally extending ledges 68 are arranged extending proximally from the transition surfaces 66. Between the longitudinal ledges 68, generally rectangular cut-outs 70, 72 have been formed. Two first oppositely positioned cut-outs 70 extend from the proximal body and a certain distance along the main body, while two second oppositely positioned cut-outs 72 extend from the proximal body to the transition surfaces. Further, two generally rectangular cut-outs 74 have been arranged at a distal end of the main body, positioned in line with the first cut-outs 70. The proximally directed surfaces of the cut-outs 74 are arranged with proximally directed and inwardly inclined tongues 76.

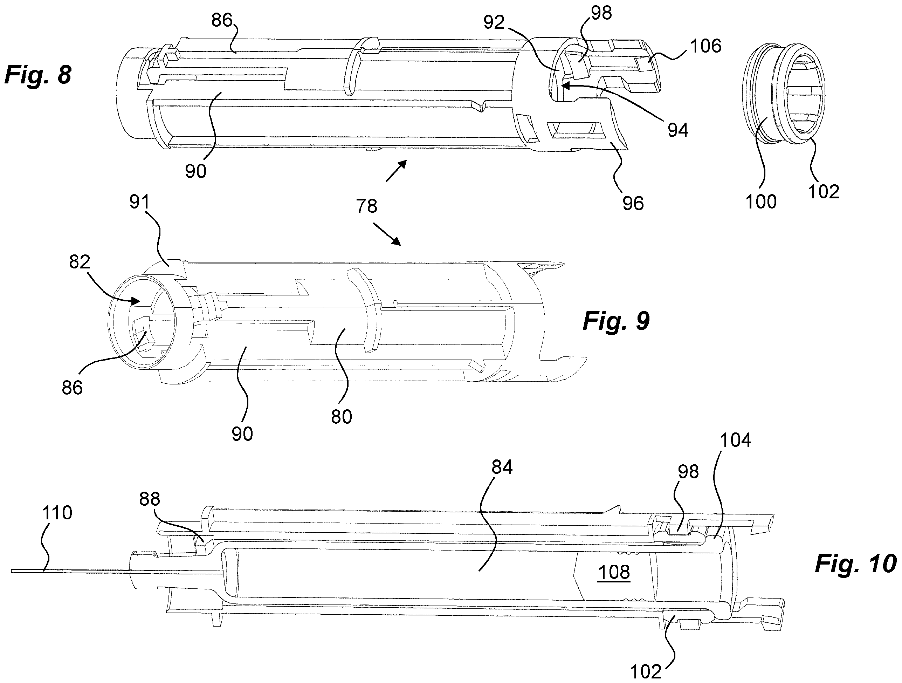

Coaxial with and inside the medicament delivery member guard 52 is a container holder 78, Figs. 8-9. The container holder 78 has a generally tubular body 80 having a proximal passage 82 through which a proximal end of a medicament container 84 may protrude. The body 80 of the medicament container holder 78 is arranged with proximally directed arms 86, which arms 86 are arranged with inwardly directed protrusions 88, which protrusions 88 are designed to be in contact with a shoulder portion of a mounted medicament container 84 as seen in Fig. 10. The body 80 of the medicament container holder 78 is further arranged with longitudinally extending ledges 90, which ledges 90 are arranged to interact with side surfaces of the first cut-outs 70 of the medicament delivery member guard 52 and in particular side surfaces at a proximal area of the first cut-outs 70. The longitudinally extending ledges 90 end in the proximal direction in transversally directed ledges 91, Fig. 9, that will act as stop ledges as will be described below.

The distal end of the medicament container holder 78 is arranged with a transversal wall 92 having a central opening 94, Fig. 8. Further, two distally directed tongues 96 are arranged at the distal end of the medicament container holder 78. The tongues 96 are provided with inwardly directed protrusions 98, which protrusions are to interact with a circumferential groove 100 of an annular holding element 102 that is designed to be in contact against a proximal surface of a flange 104 of the medicament container as seen in Fig. 10. The annular holding element 102 is preferably made in a resilient material such as rubber. The distally directed tongues 96 are further arranged with inwardly directed wedge-shaped protrusions 106. The medicament container is further provided with a movable stopper 108 of a resilient material and has a medicament delivery member at a proximal end, in the embodiment shown an attached injection needle 110. The medicament delivery member is protected by a medicament delivery member shield 111, Fig. 1, that in the embodiment shown is a rigid needle shield or RNS.

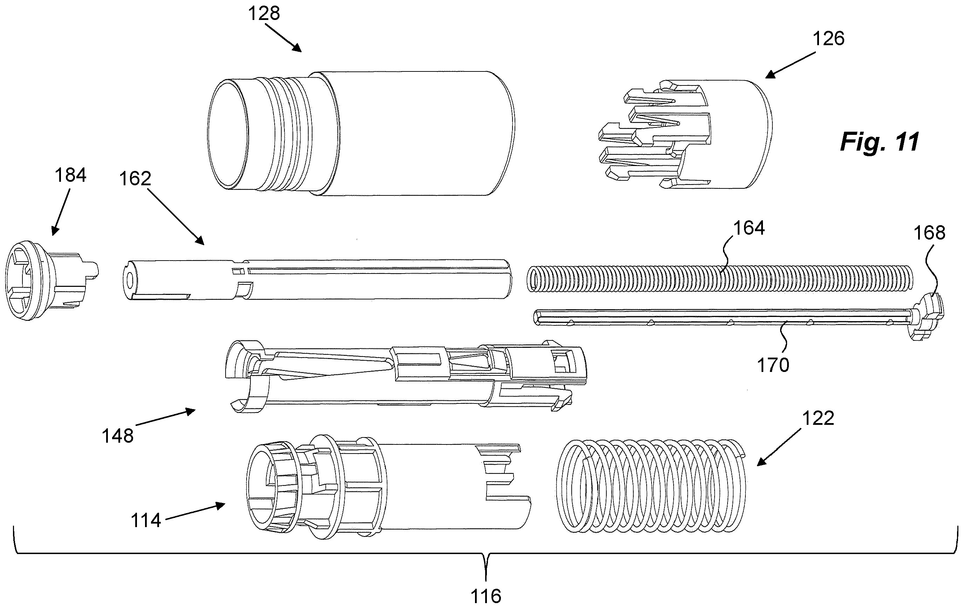

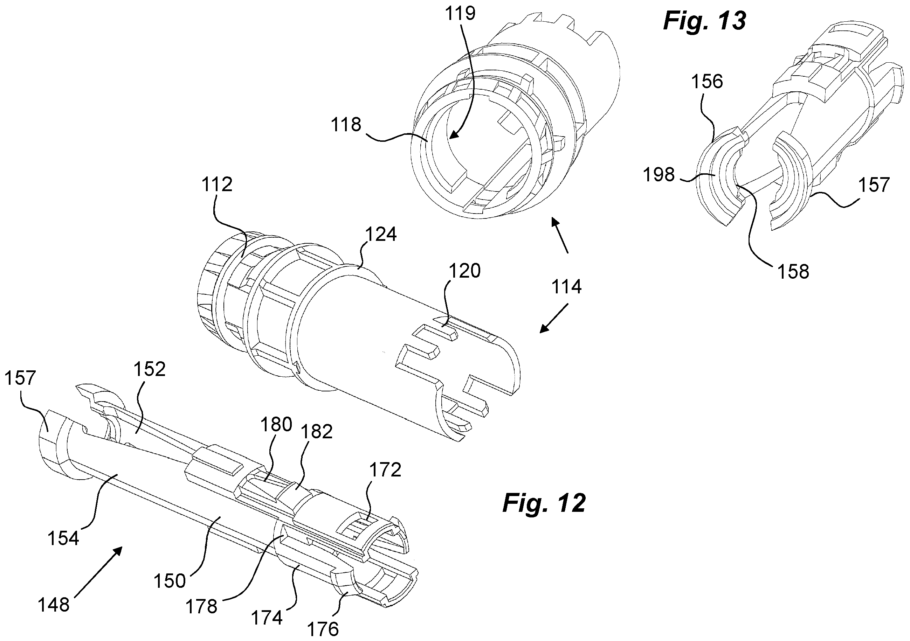

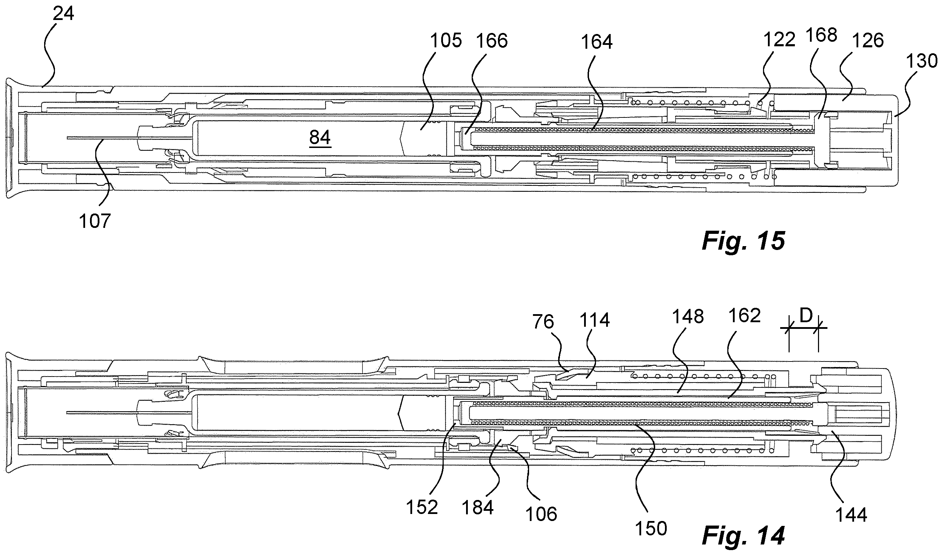

The inwardly inclined tongues 76 of the distal area of the medicament delivery member guard 52 are designed to engage with an annular ledge 112 of a proximal area of an actuator sleeve 114 comprised in a power pack 116, Figs. 11 to 13, of the medicament delivery device such that the medicament delivery member guard 52 is connected to the actuator sleeve 114 so that they are slidable together, Fig. 14. The actuator sleeve is arranged with a proximally directed ledge 118 as well as a distally directed ledge 119 on its inner surface, Fig. 13. The distal area of the actuator sleeve 114 is provided with a number of distally directed tongues 120 that are flexible in the general radial direction, the function of which will be described below.

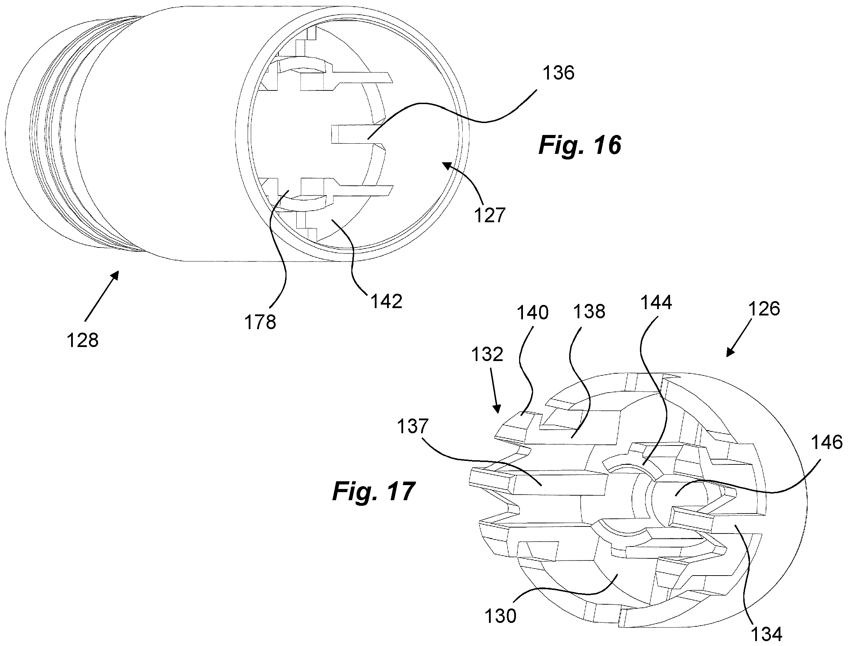

The actuator sleeve 114 and thus the medicament delivery member guard 52 is urged in the proximal direction by an actuator sleeve spring 122 that is arranged coaxial and outside the actuator sleeve 114 between an annular ledge 124 of the actuator sleeve 114 and a proximally directed surface of a manually operable activator 126, which activator 126 is protruding out of a distal passage 127 of a generally tubular power pack housing 128, Fig. 16. The activator 126 is in the embodiment shown designed as a push button having a transversal end wall 130, Figs. 15 and 16, with a distal surface acting as contact surface for a finger of a user. The push button 126 is arranged with proximally directed sections 132, Fig. 17. The outer surface of the sections 132 are provided with longitudinally extending ledges 134 that fit into grooves 136 of the power pack housing 128. Further the inner surfaces of the sections 132 are also provided with longitudinally extending ledges 137, the function of which will be explained below.

The proximal ends of the sections 132 are further provided with two arms 138 on each side of the sections 132, wherein the arms 138 are flexible in directions transversal to the sections. The arms 138 are arranged with outwardly directed ledges 140 that are designed to interact with wall sections 142, Fig. 16, of the power pack housing 128. On a proximal surface of the end wall 130 a proximally extending post 144 is arranged. The post 144 is provided with two oppositely positioned rectangular shaped slits 146.

Coaxial with and inside the actuator sleeve 114 is an actuator 148, Figs. 12, 13 and 14. It comprises a generally tubular body 150. The proximal part of the body 150 is arranged with two oppositely arranged slits 152 so that two proximally directed arms 154 are formed, wherein the slits 152 are widened in the proximal direction, creating a good flexing characteristics in the generally radial direction of the arms 154. The outer ends of the arms 154 are arranged with outwardly directed arc-shaped elements 156, Fig. 13, where the outwardly directed arc-shaped elements 156 are provided with outer surfaces 157 that are in contact with the inner surface of the actuator sleeve 114. Further, the outer ends of the arms 154 are also arranged with inwardly directed, generally arc-shaped, protrusions 158.

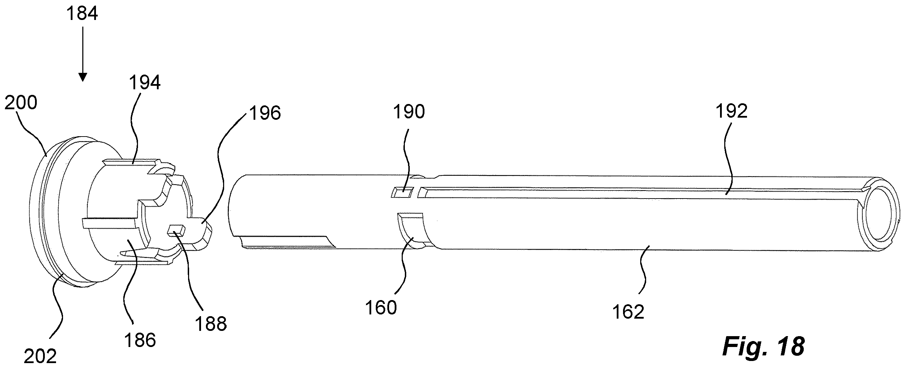

The protrusions 158 are intended to fit into recesses 160, Fig. 18, on an outer surface of a generally elongated tubular plunger rod 162. Inside the plunger rod 162 a drive spring 164 is arranged, Figs. 11 and 15, between a proximal end wall 166 of the plunger rod 162 and a transversal beam 168 on a distal end of an elongated spring guide rod 170 that runs inside the drive spring 164. The free ends of the transversal beam 168 act as fastening elements and are fitting into cut-outs 172 on a distal part of the actuator 148, Fig. 12. The transversal beam 168 further fits into the slits 146 of the post 144 of the push button 126, Fig. 16. The actuator 148 is further arranged with distally directed first arms 174 that are flexible in a generally radial direction, Fig. 12. The free ends of the arms 174 are arranged with outwardly directed ledges 176 having a slight inclination in relation to the transversal direction. The arms 174 fit into cut-outs 178 in the wall section 142 of the power pack housing 128, Fig 17, wherein the ledges 176 of the arms 174 are to interact with distally directed surfaces of the wall section 142.

On the side of the arms 174 distally directed ledges 179 are provided, which will interact with the wall sections 142 of the power pack housing 128 as will be described. The actuator 148 is also provided with second distally directed arms 180 that in the embodiment shown are oriented 90 degrees in relation to the first arms along the extension of the actuator 148. The second arms 180 are flexible in the general radial direction and are provided with outwardly directed wedge-shaped ledges 182 at the free ends. The second arms 180 are intended to interact with the distally directed tongues 120 of the actuator sleeve 114 as will be described below.

The power pack 116 is further arranged with a driver 184 having a generally tubular body 186, Fig. 18. The inner surface of the body is arranged with inwardly protrusions 188, which protrusions 188 are designed to fit into recesses 190 on the outer surface of the plunger rod 162 adjacent the recesses 160 for the protrusions 158 of the actuator 148. The plunger rod 162 is further arranged with longitudinally extending grooves 192 that are aligned with the recesses 190 and which grooves 192 extend from a distal end of the plunger and end a certain distance from the recesses 190 as seen in Fig. 18. The outer surface of the body 186 is further arranged with ribs 194, which ribs 194 are in contact with inner surfaces of the arc-shaped elements 156 of the actuator 148. The distal end surface of the body 186 is provided with distally directed protrusions 196 that fit into the slits 152 of the actuator 148. The other distal end surfaces of the body 186 are in contact with proximally directed surfaces 198 of the actuator 148. Moreover, the proximal part of the body 186 has a somewhat enlarged diameter, having an outer surface 200 that is somewhat inclined and with a distally directed circumferential ledge 202. The proximal part of the body 186 is designed to fit into the distal area of the medicament container holder 78 with its inwardly directed protrusions 106 in contact with the circumferential ledge 202, connecting the driver 184 with the medicament container holder 78.

The device is intended to be used as follows. When delivered to a user, the medicament delivery device is provided with a medicament delivery member in the form of a syringe 110 placed in the medicament container holder 78. The drive spring 164 is tensioned inside the plunger rod 162 and is held in this tensioned state by the inwardly protrusions 158 of the arms 154 fitting into the recesses 160 of the plunger rod 162, and wherein the arms 154 are held immobile by the actuator sleeve 114 being in contact with the arc-shaped elements 156 of the arms 154, Figs. 14 and 15.

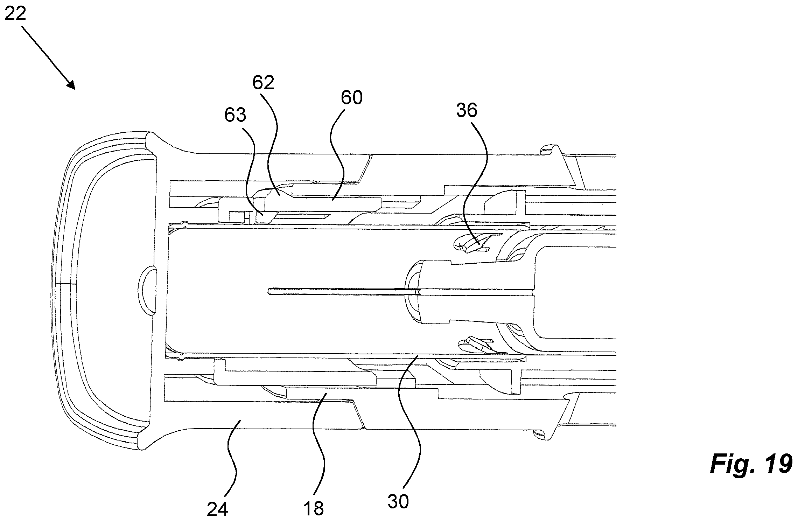

The medicament delivery member guard 52 is held stationary even if the medicament delivery device is dropped due to that the inwardly protrusions 62 of the tongue 60 of the medicament delivery member guard 52 is in contact with an outer surface of the medicament delivery member shield remover 30 so that it cannot flex inwards while the outwardly directed protrusion 62 is abutting a proximal end surface of the housing, Fig. 19. Thus the medicament delivery member guard 52 is prevented from moving in the distal direction. Further, the push button 126 may be depressed but will not affect anything in this initial positon. As seen in Fig. 14, there is a distance D between the proximal end of the post 144 of the push button 126 and the distal end of the plunger rod 162. Thus, when the push button 126 is pressed into the housing against the force of the actuator sleeve spring 122, it will not come in contact with the plunger rod 162. Thus the medicament delivery device cannot be prematurely activated in the storage position.

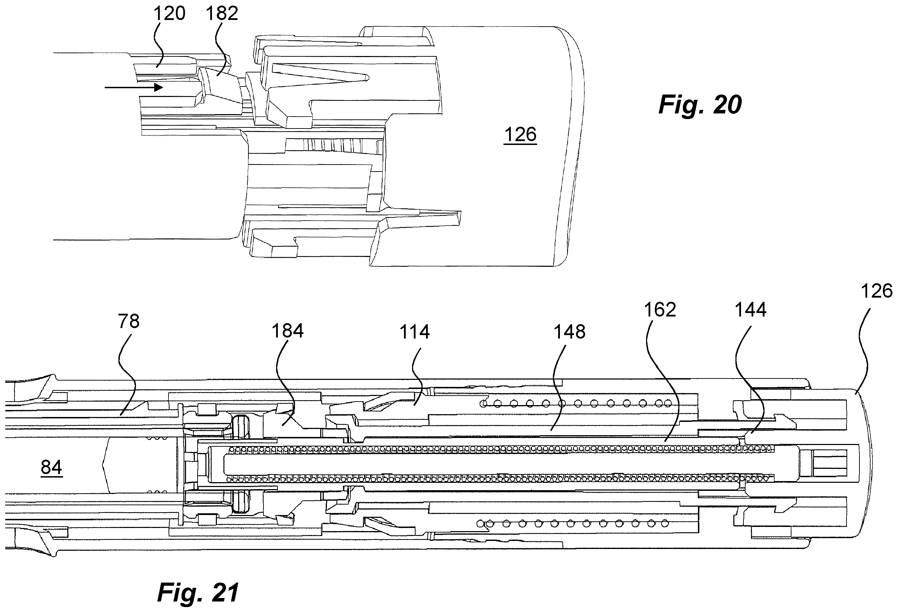

When the medicament delivery device is to be used, the user pulls the safety cap 22 off the proximal end of the medicament delivery device. This will cause the tongues 36 of the medicament delivery member shield remover 30 to grip into the material of the medicament delivery member shield 111, thereby also pulling the medicament delivery member shield 111 off the medicament delivery member. The proximal end of the device is then pressed against a dose delivery site. This causes the medicament delivery member guard 52 to move inside the housing 10 in the distal direction. Since the medicament delivery member guard 52 is connected to the actuator sleeve 114, the latter will also move distally in relation to the rest of the medicament delivery device. The distally directed tongues 120 of the actuator sleeve 114 are in line with the wedge-shaped ledges 182 of the second arms 180 of the actuator 148, wherein also the actuator 148 is moved in the distal direction, Fig. 20.

Further since the actuator 148 is connected to the plunger rod 162 via the protrusions 158 engaging the recesses 160 of the plunger rod 162, the plunger rod 160 will also move distally. Moreover, since the driver 184 is connected to the plunger rod 162 via the protrusions 188 engaging the recesses 190, the driver 184 will also move distally. In addition, since the driver 184 is connected to the medicament container holder 78 via its protrusions 106 engaging the ledge 202 of the driver 184, the container holder 78 with the container 84 will also move distally. The movement of all the mentioned components is done against the force of the actuator sleeve spring 122 and if the medicament delivery device should now be removed from the dose delivery site, all components will move back to their initial positions due to the spring force. Further, due to the movement of all the above components together with plunger rod 162, the distal end of the plunger rod 162 has now moved the distance D and is now adjacent the proximal end of the post 144 of the push button, as seen in Fig. 21.

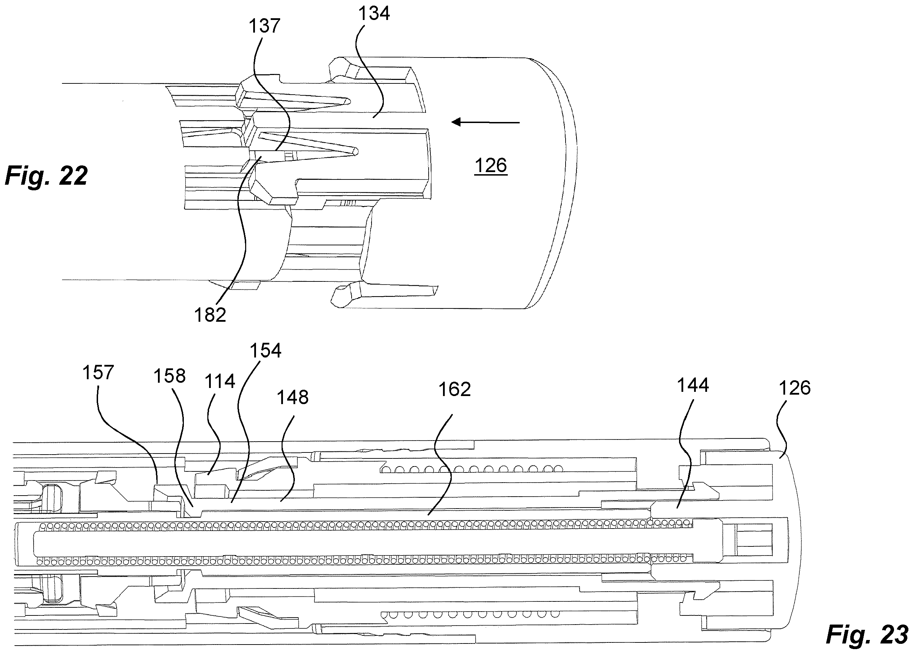

The user can now press on the push button 126 at the distal end of the medicament delivery device, causing the push button 126 to move in the proximal direction inside the housing. This will cause the second arms 180 to be pressed inwards by the longitudinal ledges 137 of the push button 126, Fig. 22, enabling the actuator 148 to be moved in the proximal direction. The movement of the push button 126 further causes the post 144 of the push button 126 to press on the distal end of the plunger rod 162, in turn causing the plunger rod 162 also to move in the proximal direction. Since the protrusions 158 of the arms 154 of the actuator 148 are positioned in the recesses 160 of the plunger rod 162, the actuator 148 will also move in the proximal direction and in particular in relation to the actuator sleeve 114. This will cause the surfaces 157 of the ends of the arms 154 of the actuator 148 to move past the edge of the actuator sleeve 114, Fig. 23.

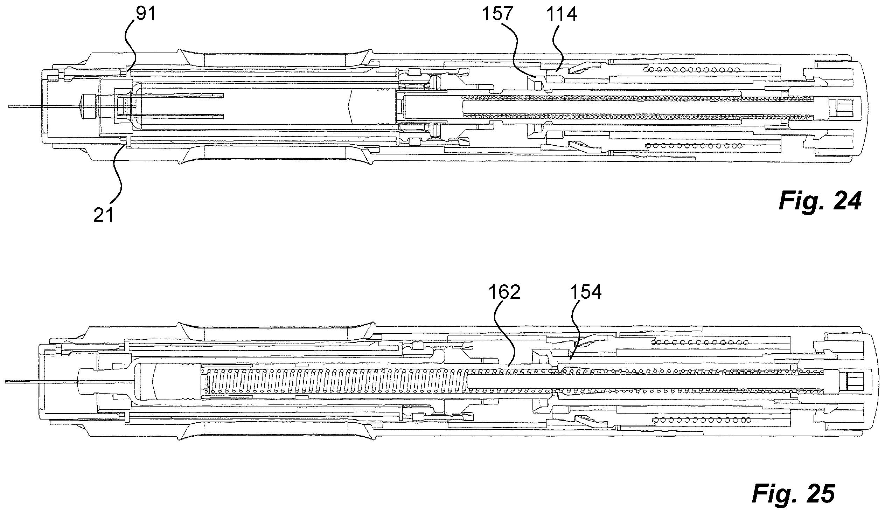

Since the actuator sleeve 114 no longer holds the arms 154, the arms 154 are free to move radially outwards whereby the plunger rod 162 is released. Due to the tensioned drive spring 164, the plunger rod 162 will be urged in the proximal direction and the inwardly directed protrusions 158 of the arms 154 of the actuator 148 will be in contact with and slide along the outer surface of the plunger rod 162. Due to the force of the drive spring 164 acting on the transversal beam 168 of the guide rod 170, which in turn is connected to the actuator 148, a force in the distal direction is acting on the actuator 148. However, since the arms 154 of the actuator 148 has flexed radially outwards, distally directed surfaces of the outwardly directed arc-shaped elements 156 will abut a proximal surface of the actuator sleeve, preventing the actuator 148 from moving in the distal direction due to the force of the drive spring 164. Further since the driver 184 is connected to the plunger rod 162 via the protrusions 188 inside the recesses 190 and since the driver 184 is connected to the medicament container holder 78, the latter is also moved proximally, Fig. 24. This will cause the medicament container 84 to move in the proximal direction, causing a penetration of the syringe 110 in the medicament delivery site.

When the medicament container holder 78 has reached its proximal end position by the proximally directed ledges 91 of the medicament container holder 78 coming in contact with the distally directed ledge 21 of the housing 10, Fig. 24, the driver 184 is also stopped and the protrusions 188 of the driver 184 are pushed out of the recesses 190 of the plunger rod 162 and the plunger rod 162 is continuing in the proximal direction through the driver 184, whereby the protrusions 188 of the driver 184 slide in the longitudinal grooves 192 of the plunger rod 162.

The plunger rod 162 now acts on the stopper 108 to push it in the proximal direction so that a dose of medicament is delivered through the syringe 110. When the stopper 108 via the plunger rod 162 is approaching its most proximal position, the distal end of the plunger rod 162 is moved out of contact with the inwardly directed protrusions 158 of the arms 154 of the actuator 148, Fig. 25. This will in turn cause the arms 154 to flex inwards whereby the arc-shaped protrusions 156 move out of contact with the proximal end of the actuator sleeve 114. The actuator 148 is now forced in the distal direction by the residual force of the drive spring 164, until the distally directed ledges 179 hit the wall section 142 of the housing of the power pack 116, causing a distinct indication of the end of the injection sequence, which indication may be both audible and tactile.

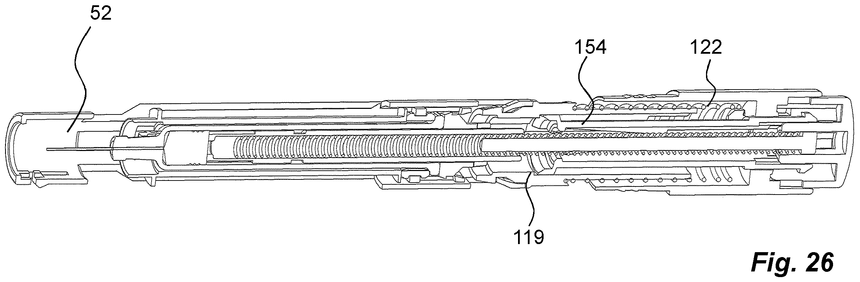

When the medicament delivery device is removed from the dose delivery site, the medicament delivery member guard 52 is pushed in the proximal direction by the actuator sleeve spring 122 so that the medicament delivery member 110 is completely covered. Further, the proximal ends of the arms 154 of the actuator 148 have passed the distally directed ledge 119 on the inner surface of the actuator sleeve 114, causing them to flex further radially outwards, Fig. 26. When the medicament delivery member guard 52 has reached its most proximal extended position it is locked in that position and prevented to be pushed in the distal direction due to that the distally directed ledge 119 of the actuator sleeve 114 will abut the proximal surfaces of the arms 154 of the actuator sleeve 114. The medicament delivery device may now be discarded.

It is to be understood that the embodiment described above and shown in the drawings is to be regarded only as a non-limiting example of the application and that it may be modified in many ways within the scope of the patent claims.

## Claims

1. Medicament delivery device comprising a housing, which housing (10) is arranged to accommodate a medicament container, further comprising a power pack arranged to exert a force on said medicament container for expelling a dose of medicament, said power pack comprising
- a plunger rod (162);
- a drive spring (164) arranged to exert a force on said plunger rod (162);
- an actuator (148) coaxial with said plunger rod (162) and arranged with holding elements (154) for releasibly holding said plunger rod (162) with said drive spring in an energized state;
- an actuator sleeve (114) coaxial with said actuator (148) and arranged to interact with said holding elements (154), said actuator sleeve (114) being movable in a longitudinal direction in relation to the actuator (148);
- a medicament delivery member guard (52) protruding from the proximal end of said housing (10) and movable in said longitudinal direction, said medicament delivery member guard (52) being connected to said actuator sleeve;
- a manually operable activator (126) arranged at a distal end of the housing (10) movable in said longitudinal direction and operably connected to said actuator (148) for releasing said plunger rod (162),
**characterised in** a locking arrangement for releasibly locking said actuator (148) to said actuator sleeve (114), such that movement of said medicament delivery member guard (52) in a distal direction when pressing the medicament delivery device against an injection site causes said actuator sleeve (114), said actuator (148) and said plunger rod (162) to move in a distal direction, and
that subsequent manual movement of said activator (126) causes a movement of said plunger rod (162) and said actuator (148) in relation to said actuator sleeve (114) in a proximal direction, causing a release of said plunger rod (162) by said actuator (148);
wherein said locking arrangement comprises a flexible member (180) on said actuator (148) provided with a proximally directed stop surface, on which stop surface a distally directed surface of said actuator sleeve (114) will act during movement in said distal direction, preventing relative movement of said actuator (148) in relation to said actuator sleeve (114) in a proximal direction, and that said activator (126) is arranged with ledges (137) acting on said flexible member (180), removing the contact between the stop surface and the surface of the actuator sleeve (114), enabling movement of said actuator (148) and said plunger rod (162).

2. Medicament delivery device according to claim 1, wherein said activator (126) further comprises a proximally directed member (144), designed to act on a distal end of said plunger rod (162) for enabling movement of said plunger rod (162) and said actuator (148) in the proximal direction.

3. Medicament delivery device according to any of the preceding claims, further comprising a driver (184) operably connected between said plunger rod (162) and a medicament container holder (78) such that movement of said plunger rod (162) in the proximal direction will cause said driver (184) and thus said medicament container holder (78) in a proximal direction, causing a penetration operation.

4. Medicament delivery device according to claim 3, wherein said driver (184) has a releasable connection to said plunger rod (162) such that said plunger rod (162) is released from said driver (184) when a penetration operation has ended, enabling said plunger rod (162) to continue in the proximal direction, acting on a stopper of the medicament and causing an injection operation.

5. Medicament delivery device according to claim 4, wherein the releasable connection comprises protrusions on an inner surface of the driver (184) cooperating with recesses (160) on the plunger rod (162).

6. Medicament delivery device according to claim 5, wherein the plunger rod (162) is arranged with a longitudinal groove (192) aligned with the recess, in which recess the protrusion of the driver (184) may slide during the injection operation.

7. Medicament delivery device according to any of the preceding claims, wherein the power pack comprises a guide rod (170) provided with fastening elements (168) at its distal end, which fastening elements (168) engage cut-outs on the actuator (148), and wherein the drive spring (164) is arranged between a proximal end wall of the plunger rod (162) and the fastening elements (168).

8. Medicament delivery device according to any of the preceding claims, wherein the holding elements (154) of the actuator (148) comprises two proximally directed arms (154) that are flexible in the generally radial direction, which arms (154) are arranged with inwardly directed arc-shaped protrusions engaging recesses (160) in said plunger rod (162), and which arm (154) are arranged with outwardly directed surfaces engaging an inner surface of said actuator sleeve (114).

## Patentansprüche

1. Medikamentenabgabevorrichtung, umfassend ein Gehäuse, wobei das Gehäuse (10) angeordnet ist, um einen Medikamentenbehälter aufzunehmen, ferner umfassend ein Netzteil, das angeordnet ist, um eine Kraft auf den Medikamentenbehälter zum Ausstoßen einer Medikamentendosis auszuüben, das Netzteil umfassend
- eine Kolbenstange (162);
- eine Antriebsfeder (164), die angeordnet ist, um eine Kraft auf die Kolbenstange (162) auszuüben;
- einen Aktuator (148), der zu der Kolbenstange (162) koaxial ist und mit Halteelementen (154) angeordnet ist, um die Kolbenstange (162) mit der Antriebsfeder in einem stromführenden Zustand lösbar zu halten;
- eine Aktuatorhülse (114), die zu dem Aktuator (148) koaxial ist und angeordnet ist, um mit den Halteelementen (154) zusammenzuwirken, wobei die Aktuatorhülse (114) in Bezug auf den Aktuator (148) in einer Längsrichtung bewegbar ist;
- einen Medikamentenabgabegliedschutz (52), der aus dem proximalen Ende des Gehäuses (10) hervorsteht und in der Längsrichtung bewegbar ist, wobei der Medikamentenabgabegliedschutz (52) mit der Aktuatorhülse verbunden ist;
- einen manuell bedienbaren Aktivator (126), der an einem distalen Ende des Gehäuses (10) angeordnet, in der Längsrichtung bewegbar und mit dem Aktuator (148) zum Freigeben der Kolbenstange (162) wirkverbunden ist,
**gekennzeichnet durch** eine Verriegelungsanordnung zum lösbaren Verriegeln des Aktuators (148) mit der Aktuatorhülse (114) derart, dass eine Bewegung des Medikamentenabgabegliedschutzes (52) in eine distale Richtung bei einem Drücken der Medikamentenabgabevorrichtung gegen eine Injektionsstelle die Aktuatorhülse (114), den Aktuator (148) und die Kolbenstange (162) veranlasst, sich in eine distale Richtung zu bewegen, und
dass eine nachfolgende manuelle Bewegung des Aktivators (126) eine Bewegung der Kolbenstange (162) und des Aktuators (148) in Bezug auf die Aktuatorhülse (114) in eine proximale Richtung veranlasst, was eine Freigabe der Kolbenstange (162) durch den Aktuator (148) veranlasst;
wobei die Verriegelungsanordnung ein flexibles Glied (180) auf dem Aktuator (148) umfasst, das mit einer proximal gerichteten Anschlagoberfläche versehen ist, auf diese Anschlagoberfläche eine distal gerichtete Oberfläche der Aktuatorhülse (114) während der Bewegung in die distale Richtung einwirkt und eine relative Bewegung des Aktuators (148) in Bezug auf die Aktuatorhülse (114) in eine proximale Richtung verhindert, und dass der Aktivator (126) mit Verstärkungsrippen (137) angeordnet ist, die auf das flexible Glied (180) einwirken und den Kontakt zwischen der Anschlagoberfläche und der Oberfläche der Aktuatorhülse (114) aufheben, was eine Bewegung des Aktuators (148) und der Kolbenstange (162) ermöglicht.

2. Medikamentenabgabevorrichtung nach Anspruch 1, wobei der Aktivator (126) ferner ein proximal gerichtetes Glied (144) umfasst, das konstruiert ist, um auf ein distales Ende der Kolbenstange (162) zum Ermöglichen der Bewegung der Kolbenstange (162) und des Aktuators (148) in der proximalen Richtung einzuwirken.

3. Medikamentenabgabevorrichtung nach einem der vorstehenden Ansprüche, ferner umfassend einen Treiber (184), der zwischen der Kolbenstange (162) und einem Medikamentenbehälterhalter (78) derart wirkverbunden ist, dass die Bewegung der Kolbenstange (162) in der proximalen Richtung den Treiber (184) und somit den Medikamentenbehälterhalter (78) in eine proximale Richtung veranlasst, was einen Penetrationsvorgang veranlasst.

4. Medikamentenabgabevorrichtung nach Anspruch 3, wobei der Treiber (184) eine lösbare Verbindung mit dem Kolbenstab (162) derart aufweist, dass der Kolbenstab (162) von dem Treiber (184) freigegeben wird, wenn ein Penetrationsvorgang beendet ist, was dem Kolbenstab (162) ermöglicht, sich in die proximale Richtung weiterzubewegen, auf einen Anschlag des Medikaments einzuwirken und einen Injektionsvorgang auszulösen.

5. Medikamentenabgabevorrichtung nach Anspruch 4, wobei die lösbare Verbindung Vorsprünge auf einer Innenoberfläche des Treibers (184) umfasst, die mit Aussparungen (160) auf der Kolbenstange (162) zusammenwirken.

6. Medikamentenabgabevorrichtung nach Anspruch 5, wobei die Kolbenstange (162) mit einer Längsnut (192) angeordnet ist, die an der Aussparung ausgerichtet ist, in diese Aussparung der Vorsprung des Treibers (184) während des Injektionsvorgangs gleiten kann.

7. Medikamentenabgabevorrichtung nach einem der vorstehenden Ansprüche, wobei das Netzteil eine Führungsstange (170) umfasst, die an ihrem distalen Ende mit Befestigungselementen (168) versehen ist, wobei die Befestigungselemente (168) in Aussparungen auf dem Aktuator (148) eingreifen, und wobei die Antriebsfeder (164) zwischen einer proximalen Endwand der Kolbenstange (162) und den Befestigungselementen (168) angeordnet ist.

8. Medikamentenabgabevorrichtung nach einem der vorstehenden Ansprüche, wobei die Halteelemente (154) des Aktuators (148) zwei proximal gerichtete Arme (154) umfassen, die in der allgemein radialen Richtung flexibel sind, wobei die Arme (154) mit nach innen gerichteten bogenförmigen Vorsprüngen angeordnet sind, die in Aussparungen (160) in der Kolbenstange (162) eingreifen, und wobei die Arme (154) mit nach außen gerichteten Oberflächen angeordnet sind, die in eine Innenoberfläche der Aktuatorhülse (114) eingreifen.

## Revendications

1. Dispositif d'administration de médicament comprenant un boîtier, lequel boîtier (10) est conçu pour accueillir un contenant de médicament, comprenant en outre un bloc d'alimentation conçu pour exercer une force sur ledit contenant de médicament pour expulser une dose de médicament, ledit bloc d'alimentation comprenant
- une tige de piston (162) ;
- un ressort d'entraînement (164) conçu pour exercer une force sur ladite tige de piston (162) ;
- un actionneur (148) coaxial avec ladite tige de piston (162) et agencé avec des éléments de maintien (154) pour maintenir de manière libérable ladite tige de piston (162) avec ledit ressort d'entraînement dans un état excité ;
- un manchon d'actionneur (114) coaxial avec ledit actionneur (148) et conçu pour interagir avec lesdits éléments de maintien (154), ledit manchon d'actionneur (114) étant mobile dans une direction longitudinale par rapport à l'actionneur (148) ;
- une protection d'élément d'administration de médicament (52) faisant saillie depuis l'extrémité proximale dudit boîtier (10) et mobile dans ladite direction longitudinale, ladite protection d'élément d'administration de médicament (52) étant reliée audit manchon d'actionneur ;
- un activateur manuel (126) disposé à une extrémité distale du boîtier (10) mobile dans ladite direction longitudinale et relié de manière opérationnelle à l'actionneur (148) pour libérer la tige de piston (162),
**caractérisé par** un agencement de verrouillage pour verrouiller de manière libérable ledit actionneur (148) audit manchon d'actionneur (114), de telle sorte qu'un mouvement de ladite protection d'élément d'administration de médicament (52) dans une direction distale lors de la pression du dispositif d'administration de médicament contre un site d'injection amène ledit manchon d'actionneur (114), ledit actionneur (148) et ladite tige de piston (162) à se déplacer dans une direction distale, et
qu'un mouvement manuel ultérieur dudit activateur (126) provoque un mouvement de ladite tige de piston (162) et dudit actionneur (148) par rapport audit manchon d'actionneur (114) dans une direction proximale, provoquant une libération de ladite tige de piston (162) par ledit actionneur (148) ;
dans lequel ledit dispositif de verrouillage comprend un élément flexible (180) sur ledit actionneur (148) pourvu d'une surface de butée orientée proximalement vers le haut, surface de butée sur laquelle agira une surface orientée distalement dudit manchon d'actionneur (114) pendant un mouvement dans ladite direction distale, empêchant un mouvement relatif dudit actionneur (148) par rapport audit manchon d'actionneur (114) dans une direction proximale, et que ledit activateur (126) est agencé avec des rebords (137) agissant sur ledit élément flexible (180), supprimant le contact entre la surface de butée et la surface du manchon d'actionneur (114), permettant un mouvement dudit actionneur (148) et de ladite tige de piston (162).

2. Dispositif d'administration de médicament selon la revendication 1, dans lequel ledit activateur (126) comprend en outre un élément dirigé proximalement (144), conçu pour agir sur une extrémité distale de ladite tige de piston (162) pour permettre un mouvement de ladite tige de piston (162) et dudit actionneur (148) dans la direction proximale.

3. Dispositif d'administration de médicament selon l'une quelconque des revendications précédentes, comprenant en outre un organe d'entraînement (184) fonctionnellement relié entre ladite tige de piston (162) et un support de récipient de médicament (78) de telle sorte qu'un mouvement de ladite tige de piston (162) dans la direction proximale entraînera ledit organe d'entraînement (184) et donc ledit support de récipient de médicament (78) dans une direction proximale, provoquant une opération de pénétration.

4. Dispositif d'administration de médicament selon la revendication 3, dans lequel ledit organe d'entraînement (184) présente une liaison amovible à ladite tige de piston (162) de telle sorte que ladite tige de piston (162) est libérée dudit organe d'entraînement (184) lorsqu'une opération de pénétration est terminée, ce qui permet à ladite tige de piston (162) de continuer dans la direction proximale, d'agir sur un bouchon du médicament et de provoquer une opération d'injection.

5. Dispositif d'administration de médicament selon la revendication 4, dans lequel la liaison amovible comprend des protubérances sur une surface interne de l'organe d'entraînement (184) coopérant avec des évidements (160) sur la tige de piston (162).

6. Dispositif d'administration de médicament selon la revendication 5, dans lequel la tige de piston (162) est agencée avec une rainure longitudinale (192) alignée sur le renfoncement, renfoncement dans lequel la protubérance de l'organe d'entraînement (184) peut glisser pendant l'opération d'injection.

7. Dispositif d'administration de médicament selon l'une quelconque des revendications précédentes, dans lequel le bloc d'alimentation comprend une tige de guidage (170) pourvue d'éléments de fixation (168) à son extrémité distale, lesquels éléments de fixation (168) entrent en prise avec des découpes sur l'actionneur (148), et dans lequel le ressort d'entraînement (164) est agencé entre une paroi d'extrémité proximale de la tige de piston (162) et les éléments de fixation (168).

8. Dispositif d'administration de médicament selon l'une quelconque des revendications précédentes, dans lequel les éléments de maintien (154) de l'actionneur (148) comprennent deux bras dirigés proximalement (154) qui sont flexibles dans la direction généralement radiale, lesquels bras (154) sont agencés avec des évidements en prise avec des protubérances en forme d'arc dirigé vers l'intérieur (160) dans ladite tige de piston (162), et lesquels bras (154) sont agencés avec des surfaces dirigées vers l'extérieur en prise avec une surface interne dudit manchon d'actionneur (114).
